# EUROPEAN PATENT SPECIFICATION

(11) **EP 2 804 670 B1**
(45) Date of publication and mention of the grant of the patent: **14.06.2017**
(21) Application number: 13700882.7
(22) Date of filing: 16.01.2013
(51) Int. Cl.: A61Q 1/02, A61K 8/11, A61K 8/19, A61K 8/26, A61K 8/37, A61K 8/30, A61Q 19/00, A61K 8/92, A61K 8/29

(54) **COSMETIC COMPOSITION COMPRISING ENCAPSULATED PIGMENTS AND REFLECTIVE PARTICLES PREDISPERSED IN AN OIL**
KOSMETIKZUSAMMENSETZUNG MIT GEKAPSELTEN PIGMENTEN UND IN EINEM ÖL VORDISPERGIERTEN REFLEKTIERENDEN PARTIKELN
COMPOSITION COSMÉTIQUE COMPRENANT DES PIGMENTS ENCAPSULÉS ET DES PARTICULES PRÉDISPERSÉES DANS UNE HUILE

(30) Priority: 17.01.2012 FR 1250456; 27.01.2012 US 201261591283 P
(43) Date of publication of application: 26.11.2014
(73) Proprietor: L'OREAL, 75008 Paris (FR)
(72) Inventor: LEPTACZ, Dominique, F-78500 Sartrouville (FR); LEBRE-LEMONNIER, Caroline, F-75005 Paris (FR); BODELIN, Sophie, F-92170 Vanves (FR)
(74) Representative: Goudet, Sylvain
(86) International application number: PCT/EP2013/050753
(87) International publication number: WO 2013/107776

(56) References cited:
- WO-A1-91/06277
- FR-A1- 2 695 558
- FR-A1- 2 949 953
- FR-A1- 2 960 774
- US-A1- 2005 276 774
- US-A1- 2007 134 180
- Anonymous: "Mirco-encapsulation", Wikipedia, 10 January 2012 (2012-01-10), XP055048349, Retrieved from the Internet: URL:http://en.wikipedia.org/w/index.php?ti tle=Special:Book&bookcmd=download&collecti on_id=6e1311ca611d43c3&writer=rl&return_to =Micro-encapsulation [retrieved on 2012-12-19]

## Description

The invention relates to a cosmetic composition for caring for and/or making up keratin materials, in particular the skin, comprising at least encapsulated pigments and reflective particles predispersed in an oil, in particular a plant oil or an ester.

Colouring substances, usually used in the cosmetics field, generally colour the composition in which they are formulated.
However, it may be desirable, for certain applications, for this coloured effect to show up only upon application and for, in other words, the composition to be, for its part, not uniformly coloured or white in colour.
In addition, users of tinted creams and care products are today looking for products which are close to care products in appearance but which make the complexion even and luminous.

Cosmetic compositions comprising pigments of which the colour effect, imperceptible in the product in its packaging, appears at the time the composition is applied to a keratin material, in particular the skin, are known in the prior art, in particular applications WO 01/35933 (Tagra Biotechnologies) and US 2005/026593. Advantageously, the switch from an initial shade of the product in its packaging, particularly remote from the colour of skin, to a colouration upon application which is provided with a natural shade, reinforces the perception by the user of personalised makeup.

Moreover, this type of composition is also appreciated by certain consumers for its playful nature.

In order to obtain this inhibition of the colour effect in the packaging, use is generally made of encapsulating pigments, also referred to as "tribosensitive", which have the particularity of disaggregating or breaking up when they are subjected to friction, crushing or shearing, thus revealing their colour at the time of application of the composition.

For the purposes of the invention, the term "encapsulated pigment" is intended to denote any pigment or mixture of pigments contained in capsules or spherules, in particular microcapsules, constituting a shell around the pigment, and the shell of which is rupturable in response to a certain pressure exerted upon application of the composition incorporating them.

The pigments are preferably encapsulated in microcapsules which are both resistant to the other raw materials present in the composition and flexible enough to be able to break under shear during application and to deliver the hoped-for colour. These encapsulated pigments are more precisely described subsequently.

However, the current compositions comprising encapsulated pigments generally exhibit in bulk in the packaging a peppery or greyish appearance, which is not very attractive for the consumer.

There remains therefore the need to provide cosmetic compositions which are attractive for the consumer, in particular cosmetic compositions which have a white or nacreous appearance when in bulk, and which make it possible, on application, to bring a colour to keratin materials, in particular to make the complexion even and luminous.

The inventors have found, surprisingly, that the use of reflective particles in a form predispersed in an oil, in a composition comprising encapsulated pigments, makes it possible to satisfy this need.

Indeed, these particles predispersed in an oil (in the form of an oily dispersion), compared with native reflective particles (in powder form), coat the microcapsules of pigments and act as a mirror, thus making it possible to reduce the visibility in bulk of the encapsulated pigments, thus giving the composition a nacreous white appearance which is more attractive for the consumer.

Applied to the skin, this composition also imparts a luminous effect to the complexion while making it even.

To the applicant's knowledge, the specific use of reflective particles predispersed in an oil, in particular a dispersion of bismuth oxychloride in ethylhexyl hydroxystearate or mica-TiO₂ in ethylhexyl hydroxystearate, which are subjects of the invention, in compositions comprising encapsulated pigments, has never been described.

A subject of the invention is therefore a cosmetic composition for caring for and/or making up keratin materials, in particular the skin, comprising, in a physiologically acceptable medium:
(i) at least one encapsulated pigment; and
(ii) at least one reflective particle predispersed in at least one oil chosen from plant oils and ester oils as defined in the claims.

In particular, the reflective particles are predispersed in at least one oil in a reflective particles/oil weight ratio of greater than or equal to 1, in particular ranging from 2 to 5, especially from 2 to 3.

The invention also relates to a cosmetic process for caring for and/or making up keratin materials, comprising the application to said keratin materials, in particular to the skin, of a composition as defined according to the invention.

It is also directed towards a process for preparing a cosmetic composition for caring for and/or making up keratin materials according to the invention, in particular the skin, comprising the addition, to a physiologically acceptable medium comprising (i) at least encapsulated pigments, of (ii) at least reflective particles in the form of a predispersion in an oil, in particular chosen from
- plant oils selected from sweet almond oil, wheat germ oil, jojoba oil, apricot oil, soya bean oil and canola oil;
- esters selected from octyldodecyl neopentanoate, caprylic/capric triglycerides, pentaerythrityl tetraisostearate, diisopropyl sebacate, C₁₂-C₁₅ alkyl benzoate, ethylhexyl ethylhexanoate and ethylhexyl hydroxystearate,
- and mixtures thereof.

The invention also relates to the use of reflective particles predispersed in an oil as defined above in a cosmetic composition comprising encapsulated pigments, as an agent intended for reducing the visibility of the encapsulated pigments in the bulk composition.

### ENCAPSULATED PIGMENTS (DYES)

The term "encapsulated dye" is intended to mean a dye which is contained in microcapsules. In particular, the dye is a pigment, in particular chosen from organic and inorganic pigments, preferably inorganic pigments.

The microcapsules can be constituted of one or more concentric layers around a core. The dye(s) can be present in the core and/or the layers around the core. The microcapsules can contain, for example, just one type of dye corresponding to just one colour, or several types of dyes.

An encapsulated dye has the advantage of being barely visible in the composition by virtue of its encapsulation, while at the same time being easily released out of the microcapsule during application.

An encapsulated pigment is different from a coated pigment commonly used in makeup products. Indeed, while coated pigments comprise a chemical coating aimed at improving the dispersion in the composition, the encapsulated pigments comprise one or more physical layer(s) forming a shell, the layers being relatively homogeneous and isolating the encapsulated pigment in a leakproof manner, such that each encapsulated pigment is well individualised in the composition, which is not the case with coated pigments.

The dyes which are encapsulated according to the invention can be chosen from pigments and lakes, and mixtures thereof.

According to one preferred mode, they are pigments.

The pigments can be chosen from surface-treated or untreated mineral pigments and organic pigments, and mixtures thereof.

The pigments may be white or coloured, and mineral and/or organic.

Among the mineral pigments are titanium dioxide, optionally surface-treated, zirconium oxide or cerium oxide, and also zinc oxide, iron (black, yellow or red) oxide or chromium oxide, manganese violet, ultramarine blue, chromium hydrate and ferric blue, and metal powders, for instance aluminium powder and copper powder.

Among the organic pigments that may be used in the invention, mention may be made of carbon black, pigments of D&C type, lakes based on cochineal carmine or on barium, strontium, calcium or aluminium, or alternatively the diketopyrrolopyrroles (DPPs) described in documents EP-A-542 669, EP-A-787 730, EP-A-787 731 and WO-A-96/08537.

According to one particular mode, they are encapsulated mineral pigments.
Thus, the composition of the invention comprises at least encapsulated pigments chosen from organic pigments and inorganic pigments, and mixtures thereof, preferably chosen from inorganic pigments. In particular, the inorganic pigment is chosen from metal oxides, such as a titanium oxide, zirconium oxide, cerium oxide, zinc oxide or iron oxide, ferric blue, chromium oxide and aluminium oxide, in particular chosen from titanium oxides and iron oxides, and mixtures thereof.

The pigments are preferably encapsulated in microcapsules which are both resistant to the other raw materials present in the composition and flexible enough to be able to break under shear during application and to thus deliver the hoped-for colour.

Thus, the encapsulated pigments are "activated" during application of the composition. In other words, after activation, the pigments give the composition applied to the keratin material a colouration which is distinct from that of the composition in the packaging.

Before application, the composition can in particular have, in its packaging, any shade, for example white or grey, associated with the other ingredients of the composition, whereas, once the pigments been activated upon application, it advantageously reveals a colouration desired by the user.

The revealing of the colour by the pigments can also be partly the result of variations in pH and in temperature between the cosmetic composition in its packaging and once it is applied to the keratin material. The bringing into contact of the encapsulated pigments, during application of the composition to the skin, with the water or other fluids present at the surface of the skin, can also promote the development of the colouration.

The mixture obtained upon application can constitute a coloured makeup and/or care composition, for example a fluid foundation, a concealer product or product for the area around the eyes, a lipstick, a liquid gloss, a face powder, an eyeliner, a mascara, an eyeshadow, a body or hair makeup product, or else a skin-colouring product.

In particular, it will be a skin makeup composition, and especially a foundation.

The shells of the microcapsules may comprise one or more layers, which are in particular concentric around the central core, and be made from materials chosen, for example, from the following materials:
- heat-meltable compounds, the melting point of which is between 30°C and 70°C, preferably between 37°C and 45°C, for example such as those described in application US 2006/0292193 A1; mention may also be made, by way of example, of the microcapsules constituted of jojoba esters, sold under the reference Florasome by the company Floratech, and described in patents US 6,432,428 and WO 2006/081351;
- polymers or copolymers, such as polyacrylates or polymethacrylates, and vinyl polymers. Mention may be made, by way of example, of the microcapsules based on an acrylate/ammonium methacrylate copolymer that are sold by Tagra Biotechnologies Ltd and are described in the publication WO 01/35933;
- polysaccharides, for instance cellulose derivatives, such as, for example, hydroxymethylcellulose, hydroxyethylcellulose, hydroxypropylcellulose and carboxymethylcellulose; starch; chitosan; algins; alginates; agars; agaroses; pectins; polypectates or carrageenans;
- polyamides;
- copolymers based on styrene/acrylate, such microcapsules being sold under the name Coloursphere by the company Créations Couleurs;
- and mixtures thereof.

Thus, the composition of the invention comprises pigments encapsulated in microcapsules chosen from:
- microcapsules constituted by jojoba esters;
- microcapsules of polymers or copolymers, such as polyacrylates or polymethacrylates, or vinyl polymers;
- microcapsules of polysaccharides, for instance cellulose derivatives, such as, for example, hydroxymethylcellulose, hydroxyethylcellulose, hydroxypropylcellulose and carboxymethylcellulose; starch; chitosan; algins; alginates; agars; agaroses; pectins; polypectates or carrageenans;
- microcapsules of polyamides;
- microcapsules of copolymers based on styrene/acrylate;
- and mixtures thereof.

According to one preferred mode, the microencapsulated pigment can be in particular contained in microcapsules based on acrylic polymers, on polysaccharides, in particular on cellulose derivatives, or mixtures thereof.

As pigments encapsulated by microcapsules made of acrylic acid and/or methacrylic acid polymers or copolymers, mention may, for example, be made of microcapsules based on a copolymer of ammonium salts of ethyl acrylate/methacrylic acid, sold by the company Tagra and described in document WO-A-01/35933. In particular, mention may be made of the encapsulated pigments sold by Tagra Biotechnologies under the name Blackcap1©, Yellowcap1© or Redcap1©, having the INCI name: iron oxide (and) titanium dioxide (and) boron nitride (and) acrylates/ammonium methacrylate copolymer (and) Triethyl citrate; mention may also be made of the encapsulated pigments sold by Tagra Biotechnologies under the name Blackcap3©, Yellowcap3© or Redcap3©, having the INCI name composed of several ingredients: titanium dioxide (and) iron oxide (and) isopropyl myristate (and) acrylates/ammonium methacrylate copolymer (and) Triethyl citrate.

As pigments encapsulated by microcapsules made of cellulose derivatives, mention may, for example, be made of spheres comprising mannitol, cellulose, hydroxypropylmethylcellulose, sold by the company Induchem under the name Unisphère©, having the INCI name: mannitol, cellulose, hydroxypropyl methylcellulose (and) titanium dioxide (and) iron oxides.

As pigments encapsulated by polysaccharide- and/or methacrylic acid polymer-based double-layer microcapsules, mention may be made, by way of example, of the encapsulated pigments described in application JP 2011-79804 and sold by Daito under the name Sugarcapsules©.

As pigments encapsulated in microcapsules made of polymers of polyester, poly(amino methacrylate), polyvinylpyrrolidone, hydroxypropylmethylcellulose or shellac, and mixtures thereof, mention may in particular be made of those described in application WO 2011/027960 by Biogenics and sold under the name Magicolor© by Biogenics.

According to one particular mode, the core may comprise the dye and be covered with layers formed from a polymeric material.

According to another particular mode, the core does not comprise any dye and is covered with layers comprising at least one dye.

The core may be covered with one or more layers.

According to one particular mode, microcapsules comprising at least 2 concentric layers around the core may be used; reference is then made to multilayer microcapsules.

Such multilayer microcapsules are described hereinafter.

### Multilayer microcapsules

According to one particular mode, the microcapsules comprise a core and at least 2 concentric layers around said core; reference is made to "multilayer" microcapsules. According to one particular mode, the outermost layer comprises a core at least one dye. The dyes are described above. According to one preferred mode, the dyes are inorganic pigments, in particular chosen from metal oxides, and especially iron oxides and titanium dioxide, and mixtures thereof.
The average size of said particles will generally range from 80µm to 800µm, in particular from 100µm to 400µm.
In particular, the average size of said particles will generally range from 50 to 1000 Mesh (approximately 400µm to 10µm), in particular from 60 to 200 Mesh (approximately 250µm to 75µm).

The microcapsules can be prepared by various methods known in the coating or encapsulating field, including pellet formation, granulation, coating, etc. By way of examples, the microcapsules may be prepared by mixing the compounds (active agents, pigments, polymers, solvents) and drying so as to form the capsules (e.g.: WO01/35933 and WO2011/027960), or by granulation and formation of the layers by spraying and drying (e.g.: FR2841155), or by means of the fluidized bed technology, used in the food and pharmaceutical industry.
For example, application WO08139053 describes the preparation of multilayer spheroids comprising a sugar core and concentric layers containing pharmaceutical active agents. The active agents are attached by impregnation or spraying or projection onto the core, then the 1^{st} layer is dried before application of a second.

### Central core

The core comprises an organic material. Preferably, the core is in a solid or crystalline form at ambient temperature. In particular, the organic material is chosen from soluble or dispersible organic materials.

According to one particular mode, the core is essentially constituted of said organic material. Preferably, the core is a sugar alcohol, preferably a monosaccharide alcohol, advantageously chosen from mannitol, erythritol, xylitol and sorbitol.

According to one preferred mode, the core is made of mannitol.

The core generally represents from 1% to 50% by weight, preferably from 5% to 30% by weight and even better from 10% to 20% by weight of microcapsules.

### Layers around the core

As described previously, the core is advantageously coated with one or more outer layers which are concentric with respect to the core, preferably at least one or more internal layer(s) and an external layer.

### Polymer

According to one particular mode, the internal and/or external layers comprise at least one polymer, preferably a hydrophilic polymer.
Said hydrophilic polymer is soluble or dispersible in water or alcoholic compounds, in particular compounds chosen from lower alcohols, glycols and polyols.
According to the invention, the term "hydrophilic polymer" is intended to mean in particular a (co)polymer capable of forming hydrogen bonds with water or said alcoholic compounds. The bonds in particular concerned are O-H, N-H and S-H bonds.
According to one particular mode, said hydrophilic polymer can swell on contact with water or said alcoholic compounds.

The hydrophilic polymer may be chosen from:
- acrylic or methacrylic acid homopolymers or copolymers or salts and esters thereof and in particular the products sold under the names Versicol F or Versicol K by the company Allied Colloid, Ultrahold 8 by the company Ciba-Geigy, and polyacrylic acids of Synthalen K type, and salts, especially sodium salts, of polyacrylic acids (corresponding to the INCI name sodium acrylate copolymer) and more particularly a crosslinked sodium polyacrylate (corresponding to the INCI name sodium acrylate copolymer (and) caprylic/capric triglyceride) sold under the name Luvigel EM by the company;
- copolymers of acrylic acid and of acrylamide sold in the form of the sodium salt thereof under the names Reten by the company Hercules, the sodium polymethacrylate sold under the name Darvan No. 7 by the company Vanderbilt, and the sodium salts of polyhydroxycarboxylic acids sold under the name Hydagen F by the company Henkel;
- polyacrylic acid/alkyl acrylate copolymers, preferably modified or unmodified carboxyvinyl polymers; the copolymers most particularly preferred according to the present invention are acrylate/C₁₀-C₃₀-alkylacrylate copolymers (INCI name: Acrylates/C₁₀₋₃₀ Alkyl acrylate Crosspolymer) such as the products sold by the company Lubrizol under the trade names Pemulen TR1, Pemulen TR2, Carbopol 1382 and Carbopol ETD 2020, and even more preferentially Pemulen TR-2;
- AMPS (polyacrylamidomethylpropanesulfonic acid partially neutralized with aqueous ammonia and highly crosslinked) sold by the company Clariant;
- AMPS/acrylamide copolymers of the type Sepigel or Simulgel sold by the company SEPPIC, especially a copolymer of INCI name Polyacrylamide (and) C13-14 Isoparaffin (and) Laureth-7;
- polyoxyethylenated AMPS/alkyl methacrylate copolymers (crosslinked or non-crosslinked) of the type Aristoflex HMS sold by the company Clariant;
- anionic, cationic, amphoteric or nonionic chitin or chitosan polymers;
- cellulose polymers, other than alkylcellulose, chosen from hydroxyethylcellulose, hydroxypropylcellulose, hydroxymethylcellulose, ethylhydroxyethylcellulose and carboxymethylcellulose, and also quaternized cellulose derivatives;
- vinyl polymers, for instance polyvinylpyrrolidones, copolymers of methyl vinyl ether and of malic anhydride, the copolymer of vinyl acetate and of crotonic acid, copolymers of vinylpyrrolidone and of vinyl acetate; copolymers of vinylpyrrolidone and of caprolactam; polyvinyl alcohol;
- optionally modified starches and derivatives;
- optionally modified polymers of natural origin, such as:
   galactomannans and derivatives thereof, such as konjac gum, gellan gum, locust bean gum, fenugreek gum, karaya gum, gum tragacanth, gum arabic, acacia gum, guar gum, hydroxypropyl guar, hydroxypropyl guar modified with sodium methylcarboxylate groups (Jaguar XC97-1, Rhodia), hydroxypropyltrimethylammonium guar chloride, and xanthan derivatives;
- alginates and carrageenans;
- glycoaminoglycans, hyaluronic acid and its derivatives;
- mucopolysaccharides, such as hyaluronic acid or chondroitin sulfates;
- and mixtures thereof.
Preferably, the hydrophilic polymer is chosen from polysaccharides and derivatives, (homo)- or (co)polymers of acrylic and methacrylic acid and esters thereof, and mixtures thereof.

Said polysaccharides and derivatives are preferably chosen from chitosan polymers, chitin polymers, cellulose polymers, starch polymers, galactomannans, alginates, carrageenans, mucopolysaccharides and derivatives thereof, and mixtures thereof.

According to one particular mode, the polysaccharides and derivatives are preferably chosen from those including one or more monosaccharides, in particular those comprising a D-glucose unit, such as starches or celluloses, and mixtures thereof.

According to one preferred mode, the polymer is starch or a derivative thereof.
The polymer may be in one or more layers and may represent from 0.5% to 20% by weight of the microcapsule, in particular from 2% to 10% by weight of the microcapsule.

### Dyes

The term "dye" refers to organic pigments such as synthetic or natural dyes FD&C or D&C, inorganic pigments such as metal oxides, or lakes such as those based on cochineal, carmine, barium, strontium, calcium or aluminium, or mixtures thereof. The pigments may be water-soluble or liposoluble.

According to one particular mode, the microcapsules comprise a mixture of 2 or more dyes, individually encapsulated in microcapsules and/or in layers of one and the same microcapsule.

Preferably, each layer of the microcapsule comprises at least one dye or a dye mixture.

The composition of the invention advantageously comprises at least 2 microcapsules of different colour.

Examples of dyes, in particular pigments, are described above.

The dyes may represent from 20% to 90% by weight, preferably from 30% to 80% by weight and in particular from 50% to 75% by weight relative to the total weight of the microcapsule.

### Lipid material

The layers may also advantageously comprise at least one lipid material. The lipid material may have amphiphilic properties, i.e. a nonpolar component and a polar component.

Said lipid materials may comprise at least one or more C₁₂-C₂₂ fatty chains, as chosen from stearic acid, palmitic acid, oleic acid, linoleic acid and linolenic acid, and mixtures thereof.

Optionally, these fatty acid chains may be the nonpolar component of the lipid material. Said lipid material is preferably chosen from phospholipids, more preferably from phosphoacylglycerols, in particular lecithins and preferably hydrogenated lecithins.

The lipid material may represent from 0.05% to 5% by weight, in particular from 0.1% to 10% by weight of the microcapsule.

By combining 2 or 3 compounds of different hardness and/or solubility in water (for example: sugar alcohols, polymers, lipid materials) in the microcapsule, it is possible to modulate the time taken by the microcapsule to break on the skin; thus, by varying the method or the intensity of application to the skin, it is possible to adjust the colouration or the degree of colour.

According to one preferred mode, the multilayers comprise at least starch as polymer and at least lecithin as lipid material.

According to one preferred mode, the microcapsule comprises a core comprising monosaccharide derivatives and a coating (layers) comprising polysaccharides (or derivatives thereof) including monosaccharides.
According to one particularly preferred mode, the microcapsules include a core comprising a polyol monosaccharide, preferably chosen from mannitol, erythritol, xylitol and sorbitol, and a coating comprising polysaccharides (or derivatives) including, as monosaccharides, D- glucose units.
According to one preferred mode, the microcapsules include 3 or 4 dyes in various layers.

The size of the microcapsules, i.e. their number-average diameter, may range, for example, from 5 to 3000 µm, preferably from 10 to 1500 µm, more preferably from 20 to 700 µm.

The percentage of pigment relative to the total weight of the encapsulated pigment (weight of the encapsulated pigment = weight of the capsule + weight of the pigment) may vary to a large extent. The amount of pigment may range, for example, from 1% to 95% by weight, preferably from 10% to 90% by weight and even more preferentially from 15% to 75% by weight relative to the total weight of the encapsulated pigment.

The composition according to the invention may contain a total amount of encapsulated pigment(s) (weight of the capsule + weight of the pigment(s)) ranging, for example, from 0.1 % to 90% by weight, preferably from 0.5% to 75% by weight and even more preferentially from 1% to 50% by weight relative to the total weight of the composition.

According to one particular embodiment, the encapsulated pigments may be present in a composition of the invention in a pigment active material content ranging from 0.5% to 20% by weight, in particular from 1 % to 15% by weight and more particularly from 2% to 12% by weight relative to the total weight of said composition.

It is also possible to use, in the same composition, several types of capsules containing encapsulated pigments and made of different encapsulating materials.

The capsules may, in addition to the pigments, contain additives such as, for example, plasticizers and/or opacifiers in order for the colour of the encapsulated pigments to be, for example, reduced.

According to one particularly preferred embodiment, the composition of the invention comprises at least pigments encapsulated by the microcapsules based on an acrylate/ammonium methacrylate copolymer, and is more particularly chosen from titanium dioxide, yellow iron oxides, red iron oxides and black iron oxides, encapsulated in microcapsules based on an acrylate/ammonium methacrylate copolymer, in particular those sold by the company Tagra Biotechnologies.

According to another particularly preferred embodiment, the composition of the invention comprises at least pigments encapsulated in microcapsules made of cellulose derivatives, in particular in microcapsules comprising mannitol, cellulose, hydroxypropylmethylcellulose, such as those sold by the company Induchem under the name Unisphère©, having the INCI name: mannitol, cellulose, hydroxypropyl methylcellulose (and) titanium dioxide (and) iron oxides.

According to another preferred mode, the composition of the invention comprises at least pigments encapsulated by microcapsules made of sugars and polysaccharides, in particular the encapsulated pigments sold by Daito under the name Sugarcapsules©.

These encapsulated pigments can be carried in anhydrous compositions, W/O or O/W or else multiple emulsions, aqueous or oily gels, etc.
Preferably, the weight ratio between the encapsulated pigments and the reflective particles will range from 3.33 x 10⁻⁴ to 10, especially from 3.33 x 10⁻⁴ to 8, in particular from 3.33 x 10⁻⁴ to 6, or even from 3.33 x 10⁻⁴ to 4.3.
In the case of the TagraCAP1 encapsulated pigments, the weight ratio between the encapsulated pigments and the reflective particles will advantageously be between 3.33 x 10⁻⁴ and 4.3.

In the case of the TagraCAP3 encapsulated pigments, the weight ratio between the encapsulated pigments and the reflective particles will advantageously be between 3.33 x 10⁻⁴ and 6.
In the case of the Sugarcapsules Magic encapsulated pigments, the weight ratio between the encapsulated pigments and the reflective particles will advantageously be between 3.33 x 10⁻⁴ and 8.

In the case of the Unispheres encapsulated pigments, the weight ratio between the encapsulated pigments and the reflective particles will advantageously be between 3.33 x 10⁻⁴ and 10.

According to one particular mode, a weight ratio between the encapsulated pigments and the reflective particles ranging from 3 to 1 will be used.

### REFLECTIVE PARTICLES DISPERSED IN AN OILY SOLVENT

The composition of the invention comprises at least reflective particles predispersed in an oily solvent.

In particular, the reflective particles are predispersed in an oily solvent in a reflective particles/oily solvent (oil) weight ratio of greater than or equal to 1, in particular ranging from 2 to 5.

Advantageously, the reflective particles have a white and/or silvery colour.

Among the reflective particles used according to the invention, mention may in particular be made of:
- bismuth oxychloride;
- nacres;
- particles with a metallic tint;
- and mixtures thereof.

Preferably, they will be chosen from bismuth oxychloride, micas coated with titanium oxide, and mixtures thereof.

According to one particularly preferred mode according to the invention, the composition comprises at least bismuth oxychloride (Cl 77163).

### Nacres

The term "nacres" should be understood as meaning iridescent or non-iridescent coloured particles of any form, especially produced by certain molluscs in their shell or alternatively synthesized, which have a colour effect via optical interference.

The nacres can be chosen from white nacres, such as mica coated with titanium or with bismuth oxychloride, coloured nacres, such as titanium-coated mica covered with iron oxides, titanium-coated mica covered with in particular ferric blue or with chromium oxide, or titanium-coated mica covered with an organic pigment of the abovementioned type, and bismuth oxychloride-based nacres.

Advantageously, it will be mica coated with titanium or a bismuth oxychloride-based nacre.

They may also be mica particles at the surface of which are superimposed at least two successive layers of metal oxides and/or of organic colorants.

Among the commercially available nacres, mention may be made of the Timica, Flamenco and Duochrome (mica-based) nacres sold by the company BASF, the Timiron nacres sold by the company Merck, the Prestige mica-based nacres sold by the company Eckart, the following nacres based on natural mica: Sunpearl from the company Sun Chemical, KTZ from the company Kobo and Sunprizma from the company Sun Chemical, the Sunshine and Sunprizma nacres based on synthetic mica sold by the company Sun Chemical, and the Timiron Synwhite nacres based on synthetic mica sold by the company Merck.
The nacres may more particularly have a yellow, pink, red, bronze, orangey, brown, gold, silver and/or coppery colour or glint.

Advantageously, the composition of the invention comprises white-coloured, bismuth oxychloride-based nacres with a silvery glint.

As nacres that may be used in the context of the present invention, mention may in particular be made of white nacres with a silvery glint, in particular sold by the company Merck under the name Xirona Silver.

### Particles with a metallic glint

Among the particles with a metallic glint, mention may be made especially of:
- particles of at least one metal and/or of at least one metal derivative,
- particles comprising a single-material or multi-material organic or inorganic substrate, at least partially coated with at least one layer with a metallic glint comprising at least one metal and/or at least one metal derivative, and
- mixtures of said particles.

The substrate may be chosen from glasses, ceramics, graphite, metal oxides, aluminas, silicas, silicates, especially aluminosilicates and borosilicates, and synthetic mica, and mixtures thereof, this list not being limiting.

Among the metals that may be present in said particles, mention may be made, for example, of Ag, Au, Cu, Al, Ni, Sn, Mg, Cr, Mo, Ti, Zr, Pt, Va, Rb, W, Zn, Ge, Te and Se, and mixtures or alloys thereof. Ag, Au, Cu, Al, Zn, Ni, Mo and Cr and mixtures or alloys thereof (for example bronzes and brasses) are preferred metals.

The term "metal derivatives" is intended to denote compounds derived from metals, especially oxides, fluorides, chlorides and sulfides.

Illustrations of these particles that may be mentioned include aluminium particles, such as those sold under the names Starbrite 1200 EAC^{®} by the company Siberline and Metalure^{®} by the company Eckart.

Mention may also be made of metal powders of copper or of alloy mixtures such as the references 2844 sold by the company Radium Bronze, metallic pigments, for instance aluminium or bronze, such as those sold under the names Rotosafe 700 from the company Eckart, the silica-coated aluminium particles sold under the name Visionnaire Bright Silver from the company Eckart, and metal alloy particles, for instance the silica-coated bronze (alloy of copper and zinc) powders sold under the name Visionaire Bright Natural Gold from the company Eckart.

They may also be particles comprising a glass substrate, such as those sold by the company Nippon Sheet Glass under the names Microglass Metashine, Xirona from the company Merck, Ronastar from the company Merck, Reflecks from the company BASF and Mirage from the company BASF.

According to one particular mode, particles having a silvery glint will be used.

By way of example of such particles, mention may, for example, be made of particles comprising a synthetic mica substrate coated with titanium dioxide, or glass particles coated either with brown iron oxide, with titanium oxide, with tin oxide or with a mixture thereof, for instance the products sold under the brand name Reflecks® by the company Engelhard.

Reflective particles are described in particular in the documents JP-A-09188830, JP-A-10158450, JP-A-10158541, JP-A-07258460 and JP-A-05017710.

Mention may also be made, still by way of example of particles with a metallic glint comprising an inorganic substrate coated with a layer of metal, of particles comprising a borosilicate substrate coated with silver also known as "white nacres".

Particles comprising a glass substrate coated with silver, in the form of platelets, are sold under the name Microglass Metashine REFSX 2025 PS by Toyal. Particles comprising a glass substrate coated with nickel/chromium/molybdenum alloy are sold under the name Crystal Star GF 550 and GF 2525 by this same company.

As an example of particles with a metallic glint which have an optionally coated metal compound at the surface, mention may be made of the particles provided under the names Metashine^{®} LE 2040 PS, Metashine^{®} 5 MC5090 PS or Metashine^{®} MC280GP (2523) by the company Nippon Sheet Glass, Spherical Silver Powder^{®} DC 100, Silver Flake^{®} JV 6 or Gold Powder^{®} A1570 by the company Engelhard, Starlight Reflections FXM^{®} by the company Energy Strategy Associates Inc, Bright Silver^{®} 1 E 0.008X0.008 by the company Meadowbrook Inventions, Ultramin^{®} (Aluminium Powder Fine Living), and Cosmetic Metallic Powder Visionnaire Bright Silver Sea^{®}, Cosmetic Metallic Powder Visionnaire Natural Gold^{®} (60314) or Cosmetic Metallic Powder Visionnaire Honey^{®} 560316° by the company Eckart.

The reflective particles with a metallic reflection can reflect the visible spectrum substantially uniformly, as is the case, for example, with particles coated with a metal such as silver or aluminium.

The reflective particles with a metallic glint may be present in a content ranging from 0.1% to 60% by weight, in particular from 1% to 30% by weight and for example from 3% to 10% by weight relative to the total weight of the composition containing them.

### Oily solvent (dispersant)

The reflective particles, in particular the bismuth oxychloride, used for the preparation of a composition according to the invention is dispersed in at least one oil as defined below (liquid mixture) and differs from reflective particles (for example: bismuth oxychloride) in powder form.

Reference will thus be made to a "predispersion" of reflective particles or of bismuth oxychloride in an oil in order to define, according to the invention, the raw material used in a process for preparing a cosmetic composition according to the invention.

The oily solvent is preferably 2-ethylhexyl hydroxystearate. Non-volatile oils which have viscosity, emollience, solubilization and colour characteristics similar to those of the abovementioned oil can also be used and are chosen from:
- plant oils selected from sweet almond oil, wheat germ oil, jojoba oil, apricot oil, soya bean oil and canola oil;
- esters selected from octyldodecyl neopentanoate, caprylic/capric triglycerides, pentaerythrityl tetraisostearate, diisopropyl sebacate, C₁₂-C₁₅ alkyl benzoate, ethylhexyl ethylhexanoate and ethylhexyl hydroxystearate,
- and mixtures thereof.

Thus, the reflective particles used according to the invention are in particular predispersed in at least one oil chosen from plant oils and esters as defined above.

According to one particular preferred mode, the reflective particles are predispersed in at least 2-ethylhexyl hydroxystearate or a mixture of 2-ethylhexyl hydroxystearate and castor oil.

According to one particular and preferred mode, the composition according to the invention thus comprises, in a physiologically acceptable medium:
(i) at least one encapsulated pigment; and
(ii) at least one reflective particle predispersed in at least one oil chosen from 2-ethylhexyl hydroxystearate.

Advantageously, the reflective particles are chosen from bismuth oxychloride and micas coated with titanium oxide, predispersed in at least 2-ethylhexyl hydroxystearate.

The reflective particle and in particular the bismuth oxychloride will generally be present in the predispersion in the presence of the oily dispersant (oil as defined above), in a content by weight ranging from 50% to 90%, in particular from 60% to 80%, and better still from 65% to 75% by weight relative to the total weight of the predispersion. The oily dispersant will consequently be present in the predispersion in a content ranging from 10% to 50% by weight, in particular from 20% to 40% by weight, and better still from 25% to 35% by weight.

According to one particular mode, use is made of a predispersion comprising a reflective particle and in particular bismuth oxychloride and an oily dispersant as defined previously, in a weight ratio ranging from 2 to 3.

According to one preferred mode, use is made of a predispersion comprising 68% to 72% by weight of bismuth oxychloride in 28% to 32% by weight of 2-ethylhexyl hydroxystearate, relative to the total weight of the predispersion, i.e. a bismuth oxychloride/oily dispersant as defined previously weight ratio of greater than or equal to 2, preferably of between 2 and 2.6.

Such a predispersion is in particular sold under the name Biron^{®} Liquid Silver by the company Merck.

The predispersion of reflective particle and in particular of bismuth oxychloride will be used in a content of raw material ranging from 0.01% to 15% by weight, preferably from 0.5% to 10% by weight relative to the total weight of said composition according to the invention.

In other words, the total content of reflective particle and in particular of bismuth oxychloride and of associated oil, as defined previously, will range from 0.01% to 15% by weight, preferably from 0.5% to 10% by weight relative to the total weight of said composition according to the invention, in particular with a reflective particle/oily dispersant (associated oil) weight ratio ranging from 2 to 3.

According to one particular mode, these values do not take into account the contents of the optional additional oils present in the final composition.

According to one preferred mode, the reflective particle and in particular the bismuth oxychloride is dispersed in at least one oil chosen from castor oil and 2-ethylhexyl hydroxystearate, more preferably 2-ethylhexyl hydroxystearate.

According to one particular and preferred mode, the composition according to the invention is in the form of a water-in-oil emulsion.

According to one particular mode, the composition according to the invention is a facial makeup composition, in particular a foundation or a complexion illuminator.

The invention also relates to a process for preparing a cosmetic composition for caring for and/or making up keratin materials according to the invention, in particular the skin, comprising the addition, to a physiologically acceptable medium comprising (i) at least one encapsulated pigment, of (ii) at least one reflective particle in the form of a predispersion in an oil as defined above, in particular chosen from 2-ethylhexyl hydroxystearate.

According to the invention, it is intended for the reflective particles to be predispersed beforehand in an oil, before integration into the fatty phase of the composition. They either exist commercially in this form of a predispersion in an oil, or a predispersion of these reflective particles in an oil is prepared, before addition to the fatty phase of the composition.

The invention is thus directed towards a preparation process in which the reflective particles in the form of a predispersion in an oil chosen from
- plant oils selected from sweet almond oil, wheat germ oil, jojoba oil, apricot oil, soya bean oil and canola oil;
- esters selected from octyldodecyl neopentanoate, caprylic/capric triglycerides, pentaerythrityl tetraisostearate, diisopropyl sebacate, C₁₂-C₁₅ alkyl benzoate, ethylhexyl ethylhexanoate and ethylhexyl hydroxystearate,
- and mixtures thereof.
The predispersion is generally added to the fatty phase of said composition.

The reflective particles predispersed in the oils and the encapsulated pigments are as defined hereinafter in the invention.
According to one preferred mode, said composition is in the form of an oil-in-water (O/W) emulsion, a water-in-oil (W/O) emulsion, or a multiple emulsion, preferably a water-in-oil (W/O) emulsion.
According to one preferred mode, said composition according to the invention also contains at least one additional ingredient chosen from silicone oils or hydrocarbon-based oils, surfactants, fillers, non-encapsulated additional colorants, and mixtures thereof.

### GALENICAL FORM

A composition of the invention must be cosmetically acceptable, i.e. it must contain a non-toxic physiologically acceptable medium that can be applied to human keratin materials.

For the purposes of the invention, the term "cosmetically acceptable" is intended to mean a composition of pleasant appearance, odour and feel.

The physiologically acceptable medium is generally adjusted to the way in which the composition must be packaged. In particular, the nature and the content of the various compounds are adjusted, for example, according to whether the composition is formulated in solid or fluid form.

The composition according to the invention may be in the form of a composition for making up and/or caring for keratin materials, in particular the skin or the lips. In particular, the composition of the invention may be a foundation, in particular to be applied to the face or the neck, a concealer product, a complexion corrector, a tinted cream, or a coloured care or makeup composition for the skin, in particular facial or bodily skin.
The compositions according to the invention may be in any of the galenical forms conventionally used for topical application, and in particular in the form of more or less viscous, fluid compositions with a creamy or pasty appearance, such as, for example, in the form of an oily solution or an oily gel, or in the form of oil-in-water (O/W) or water-in-oil (W/O) or multiple (triple: W/O/W or O/W/O) emulsions.
According to one particular preferred mode, the composition of the invention is in the form of a fluid composition comprising at least one oily fatty phase, in particular in the form of an oil-in-water (O/W) emulsion, a water-in-oil (W/O) emulsion or a multiple emulsion, preferably a water-in-oil (W/O) emulsion.

These compositions are prepared according to the usual methods.

In addition, the compositions according to the invention may be solid or more or less fluid and may have the appearance of a cream, an ointment, a milk, a lotion, a serum, a paste, a foam (without propellant gas) or a stick.

According to one preferred embodiment, a composition may be in the form of an emulsion or an emulsified gel.

### Aqueous phase

A composition according to the invention may comprise an aqueous phase.

The aqueous phase comprises water. A water that is suitable for use in the invention may be a floral water such as cornflower water and/or a mineral water such as Vittel water, Lucas water or La Roche Posay water and/or a spring water.

The aqueous phase may also comprise organic solvents which are water-miscible (at ambient temperature: 20-25°C), for instance monoalcohols containing from 2 to 6 carbon atoms, such as ethanol or isopropanol; polyols especially containing from 2 to 20 carbon atoms, preferably containing from 2 to 10 carbon atoms and preferentially containing from 2 to 6 carbon atoms, such as glycerol, propylene glycol, butylene glycol, pentylene glycol, hexylene glycol, dipropylene glycol or diethylene glycol; glycol ethers (especially containing from 3 to 16 carbon atoms) such as mono-, di- or tripropylene glycol (C₁-C₄)alkyl ethers, mono-, di- or triethylene glycol (C₁-C₄)alkyl ethers, and mixtures thereof.

The aqueous phase may also comprise stabilizers, for example sodium chloride, magnesium dichloride or magnesium sulfate.

The aqueous phase may also comprise any water-soluble or water-dispersible compound that is compatible with an aqueous phase, such as gelling agents, film-forming polymers, thickeners or surfactants, and mixtures thereof.

In particular, a composition of the invention may comprise an aqueous phase in a content ranging from 1% to 80% by weight, in particular from 5% to 50% and more particularly from 10% to 45% by weight relative to the total weight of the composition.

According to another embodiment, a composition may comprise less than 3% by weight of water, relative to the total weight of the composition, and in particular less than 2%, especially less than 1% by weight of water relative to the total weight of the composition, the water not being added during the preparation of the composition, but corresponding to the residual water introduced by the ingredients mixed in, or it is even free of water; it will be an anhydrous composition.

### Fatty phase

A cosmetic composition in accordance with the present invention may comprise at least one liquid fatty phase.

In particular, a composition of the invention may comprise at least one oil as mentioned hereinafter.

The term "oil" is intended to mean any fatty substance that is in liquid form at ambient temperature (20-25°C) and at atmospheric pressure.

A composition of the invention may comprise a liquid fatty phase in a content ranging from 1% to 90%, in particular from 5% to 80%, in particular from 10% to 70% and more particularly from 20% to 50% by weight relative to the total weight of the composition.

The oily phase that is suitable for preparing the cosmetic compositions according to the invention may comprise hydrocarbon-based oils, silicone oils, fluoro oils or non-fluoro oils, or mixtures thereof.

The oils may be volatile or non-volatile.

They may be of animal, plant, mineral or synthetic origin.

For the purposes of the present invention, the term "volatile oil" is intended to mean an oil (or non-aqueous medium) that is capable of evaporating on contact with the skin in less than one hour, at ambient temperature and at atmospheric pressure. The volatile oil is a volatile cosmetic oil, which is liquid at ambient temperature, especially having a non-zero vapour pressure, at ambient temperature and at atmospheric pressure, in particular having a vapour pressure ranging from 0.13 Pa to 40 000 Pa (10⁻³ to 300 mmHg), preferably ranging from 1.3 Pa to 13 000 Pa (0.01 to 100 mmHg), and preferentially ranging from 1.3 Pa to 1300 Pa (0.01 to 10 mmHg).

For the purposes of the present invention, the term "non-volatile oil" is intended to mean an oil with a vapour pressure of less than 0.13 Pa.

For the purposes of the present invention, the term "silicone oil" is intended to mean an oil comprising at least one silicon atom, and in particular at least one Si-O group.

The term "fluoro oil" is intended to mean an oil comprising at least one fluorine atom.

The term "hydrocarbon-based oil" is intended to mean an oil mainly containing hydrogen and carbon atoms.

The oils may optionally comprise oxygen, nitrogen, sulfur and/or phosphorus atoms, for example in the form of hydroxyl or acid radicals.

### Volatile oils

The volatile oils may be chosen from hydrocarbon-based oils containing from 8 to 16 carbon atoms, and in particular branched C₈-C₁₆ alkanes (also known as isoparaffins), for instance isododecane (also known as 2,2,4,4,6-pentamethylheptane), isodecane, isohexadecane and, for example, the oils sold under the trade names Isopar^{®} or Permethyl^{®}.

Volatile oils that may also be used include volatile silicones, for instance volatile linear or cyclic silicone oils, especially those with a viscosity ≤ 8 centistokes (cSt) (8 × 10⁻⁶ m²/s), and especially containing from 2 to 10 silicon atoms and in particular from 2 to 7 silicon atoms, these silicones optionally comprising alkyl or alkoxy groups containing from 1 to 10 carbon atoms. As volatile silicone oils that may be used in the invention, mention may be made in particular of dimethicones with a viscosity ranging from 1 to 6 cSt, in particular from 2 to 6 cSt, octamethylcyclotetrasiloxane, decamethylcyclopentasiloxane, dodecamethylcyclohexasiloxane, heptamethylhexyltrisiloxane, heptamethyloctyltrisiloxane, hexamethyldisiloxane, octamethyltrisiloxane, decamethyltetrasiloxane and dodecamethylpentasiloxane, and mixtures thereof.

Volatile fluoro oils such as nonafluoromethoxybutane or perfluoromethylcyclopentane, and mixtures thereof, may also be used.

According to one embodiment, a composition of the invention may comprise from 1% to 80% by weight, or even from 5% to 70% by weight, or even from 10% to 60% by weight and especially from 15% to 50% by weight of volatile oil relative to the total weight of the composition.

### Non-volatile oils

The non-volatile oils may be chosen in particular from non-volatile hydrocarbon-based, fluoro and/or silicone oils.

Non-volatile hydrocarbon-based oils that may in particular be mentioned include:
- hydrocarbon-based oils of animal origin, such as perhydrosqualene,
- hydrocarbon-based oils of plant origin, such as phytostearyl esters, such as phytostearyl oleate, phytostearyl isostearate and lauroyl/octyldodecyl/phytostearyl glutamate (Ajinomoto, Eldew PS203), triglycerides formed from fatty acid esters and glycerol, in particular in which the fatty acids may have chain lengths ranging from C₄ to C₃₆ and especially from C₁₈ to C₃₆, these oils possibly being linear or branched, and saturated or unsaturated; these oils may especially be heptanoic or octanoic triglycerides, shea oil, alfalfa oil, poppy oil, winter squash oil, millet oil, barley oil, quinoa oil, rye oil, candlenut oil, passionflower oil, shea butter, aloe vera oil, sweet almond oil, peach stone oil, groundnut oil, argan oil, avocado oil, baobab oil, borage oil, broccoli oil, calendula oil, camelina oil, canola oil, carrot oil, safflower oil, flaxseed oil, rapeseed oil, cottonseed oil, coconut oil, marrow seed oil, wheatgerm oil, jojoba oil, lily oil, macadamia oil, corn oil, meadowfoam oil, St John's Wort oil, monoi oil, hazelnut oil, apricot kernel oil, walnut oil, olive oil, evening primrose oil, palm oil, blackcurrant seed oil, kiwi seed oil, grapeseed oil, pistachio oil, winter squash oil, pumpkin oil, quinoa oil, musk rose oil, sesame oil, soybean oil, sunflower oil, castor oil and watermelon oil, and mixtures thereof, or alternatively caprylic/capric acid triglycerides, such as those sold by the company Stéarineries Dubois or those sold under the names Miglyol 810^{®}, 812^{®} and 818^{®} by the company Dynamit Nobel,
- linear or branched hydrocarbons of mineral or synthetic origin, such as liquid paraffins and derivatives thereof, petroleum jelly, polydecenes, polybutenes, hydrogenated polyisobutene such as Parleam, and squalane,
- synthetic ethers containing from 10 to 40 carbon atoms,
- synthetic esters, for instance oils of formula R₁COOR₂, in which R₁ represents a linear or branched fatty acid residue containing from 1 to 40 carbon atoms, and R₂ represents a hydrocarbon-based chain that is especially branched, containing from 1 to 40 carbon atoms, provided that R₁ + R₂ ≥ 10. The esters may be chosen especially from fatty acid esters and of alcohols, for instance cetostearyl octanoate, isopropyl alcohol esters, such as isopropyl myristate, isopropyl palmitate, ethyl palmitate, 2-ethylhexyl palmitate, isopropyl stearate, isopropyl isostearate, isostearyl isostearate, octyl stearate, hydroxylated esters, for instance isostearyl lactate, octyl hydroxystearate, diisopropyl adipate, heptanoates, and especially isostearyl heptanoate, alcohol or polyalcohol octanoates, decanoates or ricinoleates, for instance propylene glycol dioctanoate, cetyl octanoate, tridecyl octanoate, 2-ethylhexyl 4-diheptanoate, 2-ethylhexyl palmitate, alkyl benzoate, polyethylene glycol diheptanoate, propylene glycol 2-diethylhexanoate, and mixtures thereof, C₁₂-C₁₅ alkyl benzoates, hexyl laurate, neopentanoic acid esters, for instance isodecyl neopentanoate, isotridecyl neopentanoate, isostearyl neopentanoate, octyldodecyl neopentanoate, isononanoic acid esters, for instance isononyl isononanoate, isotridecyl isononanoate, octyl isononanoate, hydroxylated esters, for instance isostearyl lactate and diisostearyl malate,
- polyol esters and pentaerythritol esters, for instance dipentaerythrityl tetrahydroxystearate/tetraisostearate,
- esters of diol dimers and of diacid dimers, such as Lusplan DD-DA5^{®} and Lusplan DD-DA7^{®} sold by the company Nippon Fine Chemical and described in patent application US 2004-175338,
- copolymers of a diol dimer and of a diacid dimer and esters thereof, such as dilinoleyl diol dimer/dilinoleic dimer copolymers and esters thereof, for instance Plandool-G,
- copolymers of polyols and of diacid dimers, and esters thereof, such as Hailuscent ISDA or the dilinoleic acid/butanediol copolymer,
- fatty alcohols that are liquid at ambient temperature, with a branched and/or unsaturated carbon-based chain containing from 12 to 26 carbon atoms, for instance 2-octyldodecanol, isostearyl alcohol, oleyl alcohol, 2-hexyldecanol, 2-butyloctanol and 2-undecylpentadecanol,
- C₁₂-C₂₂ higher fatty acids, such as oleic acid, linoleic acid or linolenic acid, and mixtures thereof,
- dialkyl carbonates, the two alkyl chains possibly being identical or different, such as dicaprylyl carbonate sold under the name Cetiol CC^{®} by Cognis,
- oils of high molar mass, in particular having a molar mass ranging from about 400 to about 10 000 g/mol, in particular from about 650 to about 10 000 g/mol, in particular from about 750 to about 7500 g/mol and more particularly ranging from about 1000 to about 5000 g/mol. As oils of high molar mass that may be used in the present invention, mention may especially be made of oils chosen from:
   - lipophilic polymers,
   - linear fatty acid esters with a total carbon number ranging from 35 to 70,
   - hydroxylated esters,
   - aromatic esters,
   - C₂₄-C₂₈ branched fatty acid or fatty alcohol esters,
   - silicone oils,
   - oils of plant origin,
   - and mixtures thereof,
- optionally partially hydrocarbon-based and/or silicone-based fluoro oils, for instance fluorosilicone oils, fluoropolyethers and fluorosilicones as described in document EP-A-847 752,
- silicone oils, for instance non-volatile linear or cyclic polydimethylsiloxanes (PDMSs); polydimethylsiloxanes comprising alkyl, alkoxy or phenyl groups, which are pendant or at the end of a silicone chain, these groups containing from 2 to 24 carbon atoms; phenyl silicones, for instance phenyl trimethicones, phenyl dimethicones, phenyl trimethylsiloxy diphenyl siloxanes, diphenyl dimethicones, diphenyl methyldiphenyl trisiloxanes and 2-phenylethyl trimethylsiloxy silicates, and
- mixtures thereof.

### Other ingredients

A composition according to the invention may also comprise additional ingredients chosen from silicone oils or hydrocarbon-based oils distinct from the oils for dispersion of the reflective particles mentioned previously, surfactants, fillers, non-encapsulated additional colorants, and mixtures thereof.

In particular, the composition may comprise, as additional colorants, titanium dioxide, lakes, dyes, pigments, nacres and mixtures thereof.

A composition according to the invention may also comprise any ingredient (adjuvant) normally used in the cosmetics industry, in particular in colouring and care compositions, such as thickeners or gelling agents, film-forming polymers, waxes, vitamins, trace elements, emollients, sequestering agents, fragrances, basifying or acidifying agents, preservatives, sunscreens, surfactants, antioxidants, cosmetic active agents, moisturisers, or mixtures thereof.

Needless to say, those skilled in the art will take care to select the optional additional compounds and/or the amount thereof such that the advantageous properties intrinsically attached to the composition in accordance with the invention are not, or are not substantially, adversely affected by the envisaged addition.

The invention is illustrated in the examples presented hereinafter by way of non-limiting illustration of the field of the invention.

Unless otherwise indicated, the values in the examples below are expressed as % by weight relative to the total weight of the composition.

### EXEMPLARY EMBODIMENTS

Several compositions were prepared according to a conventional protocol for formulation of emulsions.

### Examples 1 to 3: Composition with encapsulated pigments (Tagra) according to the invention and comparative examples (without oily dispersion of reflective particles)

The following 3 water-in-oil W/O emulsions were prepared:
- Example 1 (invention) comprising encapsulated pigments and bismuth oxychloride dispersed in ethylhexyl hydroxystearate;
- Example 2 (comparative) equivalent to Example 1, but without bismuth oxychloride dispersed in ethylhexyl hydroxystearate;
- Example 3 (comparative) equivalent to Example 1, but with bismuth oxychloride in conventional form (powder) not predispersed in ethylhexyl hydroxystearate (at equivalent active material content).

| | Ex.1 | Ex. 2 | Ex.3 |
|---|---|---|---|
| | (invention) | (comparative) | (comparative) |
| Chemical name (INCI name) | % | % | % |
| MAGNESIUM SULFATE. 7 H₂O | 0.70 | 0.70 | 0.70 |
| DISTEARYLDIMETHYLAMM ONIUM- MODIFIED | 0.80 | 0.80 | 0.80 |
| HECTORITE | | | |
| TALC: MICRONIZED MAGNESIUM SILICATE (PARTICLE SIZE: 5 MICRONS) (Cl: 77718) | 0.50 | 0.50 | 0.50 |
| **MICROENCAPSULATED YELLOW IRON OXIDE (YELLOWCAP1 from TAGRA BIOTECHNOLOGIES)** | **2.00** | **2.00** | **2.00** |
| **MICROENCAPSULATED RED IRON OXIDE (REDCAP1 from TAGRA BIOTECHNOLOGIES)** | **0.71** | **0.71** | **0.71** |
| **MICROENCAPSULATED BLACK IRON OXIDE (BLACKCAP1 from TAGRA BIOTECHNOLOGIES)** | **0.29** | **0.29** | **0.29** |
| REFINED PLANT PERHYDROSQUALENE | 1.00 | 1.00 | 1.00 |
| 2-ETHYLHEXYL 4-METHOXYCINNAMATE | 3.00 | 3.00 | 3.00 |
| **BISMUTH OXYCHLORIDE AND ETHYLHEXYL HYDROXYSTEARATE (TIMIRON LIQUID SILVER from Merck)** | **3.00** | **0** | **0** |
| BISMUTH OXYCHLORIDE (PEARL 2600 UVS from FARMAQUIMIA) | 0 | 0 | 2.10 |
| NYLON-12 MICROSPHERES (PARTICLE SIZE: 5 MICRONS) | 0.50 | 0.50 | 0.50 |
| PHENYLTRIMETHYLSILOXY TRISILOXANE (VISCOSITY: 20 cSt - MW: 372) | 2.00 | 2.00 | 2.00 |
| POLYDIMETHYLSILOXANE COMPRISING ALPHA,OMEGA-OXYETHYLENATED/- OXYPROPYLENATED | 1.00 | 1.00 | 1.00 |
| GROUPS IN SOLUTION IN CYCLOPENTASILOXANE | | | |
| OXYETHYLENATED POLYDIMETHYLSILOXANE (DP: 70 - VISCOSITY: 500 cSt) | 2.00 | 2.00 | 2.00 |
| POLYDIMETHYLSILOXANE 2 cSt | 28.98 | 31.98 | 29.88 |
| 1,3-BUTYLENE GLYCOL | 3.00 | 3.00 | 3.00 |
| DENATURED 96° ETHYL ALCOHOL | 5.00 | 5.00 | 5.00 |
| MICROBIOLOGICALLY CLEAN DEIONIZED WATER | 45.52 | 45.52 | 45.52 |

### Procedure:

The aqueous phase (water, butylene glycol, magnesium sulfate) and the oily phase (silicone surfactants, oils, fillers) are prepared separately.

The aqueous phase and the oily phase are mixed with stirring in a Moritz apparatus until homogenization.

The bismuth oxychloride dispersed in ethylhexyl hydroxystearate (or in powder form in Example 3) is added with stirring in a Moritz apparatus until homogenization.

The denatured alcohol is then added with stirring in a Moritz apparatus.

Finally, the encapsulated pigments are added with gentle stirring in a Rayneri apparatus until homogenization.

### Observations regarding the bulk composition and the makeup result after application to the skin

In the pot (3 kg), the composition of Example 1 has a nacreous light violet appearance, the encapsulated pigments being predominantly coated with the predispersion of bismuth oxychloride.

On the hand, i.e. 0.3 g of product, the composition has a nacreous white-grey appearance, the encapsulated pigments being predominantly coated with the predispersion of bismuth oxychloride.

Applied to and spread on the skin, this composition imparts a luminous effect to the complexion while making it even.

The compositions of Example 2 (without dispersion of bismuth oxychloride) or of Example 3 (with bismuth oxychloride in powder form) have a dark greyish appearance, in the pot or on the hand.

Applied to and spread on the skin, these compositions of Example 2 or of Example 3 impart little or no luminous effect to the complexion.

### Examples 4 and 5: Compositions according to the invention with pigments or additional dyes in the external phase

Two W/O emulsions according to the invention comprising, in addition to the encapsulated pigments, additional colorants were prepared.

| | Ex.4 | Ex. 5 |
|---|---|---|
| Chemical name (INCI name) | % | % |
| MAGNESIUM SULFATE. 7 H₂O | 0.70 | 0.70 |
| DISTEARYLDIMETHYLAMMONIUM-MODIFIED HECTORITE | 0.80 | 0.80 |
| TALC: MICRONIZED MAGNESIUM SILICATE (PARTICLE SIZE: 5 MICRONS) (Cl: 77718) | 0.50 | 0.50 |
| SYNTHETIC MICA (FLUORPHLOGOPITE) (SIZE 10-50 µM) | 0.10 | 0.20 |
| ANATASE TITANIUM OXIDE COATED WITH ALUMINIUM STEAROYL GLUTAMATE (97/3) (Cl: 77891) | 0.90 | 1.8 |
| MIXTURE SYNTHETIC TITANIUM DIOXIDE LITHOL RUBINE BCA, POLYMETHYLDROGENSILOXANE (TR-50FG from TODA KOGYO) | 0 | 0.06 |
| **MICROENCAPSULATED YELLOW IRON OXIDE (YELLOWCAP1 from TAGRA BIOTECHNOLOGIES)** | **2.14** | **2** |
| **MICROENCAPSULATED RED IRON OXIDE (REDCAP1 from TAGRA BIOTECHNOLOGIES)** | **0.76** | **0.71** |
| **MICROENCAPSULATED BLACK IRON OXIDE (BLACKCAP1 from TAGRA BIOTECHNOLOGIES)** | **0.2** | **0.29** |
| REFINED PLANT PERHYDROSQUALENE | 1.00 | 1.00 |
| 2-ETHYLHEXYL 4-METHOXYCINNAMATE | 3.00 | 3.00 |
| **BISMUTH OXYCHLORIDE AND ETHYLHEXYL HYDROXYSTEARATE (TIMIRON LIQUID SILVER from Merck)** | **3.00** | **3.00** |
| NYLON-12 MICROSPHERES (PARTICLE SIZE: 5 MICRONS) | 0.50 | 0.50 |
| PHENYLTRIMETHYLSILOXYTRISILOXANE (VISCOSITY: 20 cSt - MW: 372) | 2.00 | 2.00 |
| POLYDIMETHYLSILOXANE COMPRISING ALPHA,OMEGA-OXYETHYLENATED / - OXYPROPYLENATED GROUPS IN SOLUTION IN CYCLOPENTASILOXANE | 1.00 | 1.00 |
| OXYETHYLENATED POLYDIMETHYLSILOXANE (DP: 70 - VISCOSITY: 500 cSt) | 2.00 | 2.00 |
| POLYDIMETHYLSILOXANE 2 cSt | 27.88 | 26.92 |
| 1,3-BUTYLENE GLYCOL | 3.00 | 3.00 |
| DENATURED 96° ETHYL ALCOHOL | 5.00 | 5.00 |
| MICROBIOLOGICALLY CLEAN DEIONIZED WATER | 45.52 | 45.52 |

### Procedure

The composition of Example 4 is prepared according to the protocol described in Example 1, the only difference being that the additional colorants are wetted beforehand with an oil and ground before integration into the fatty phase, at the beginning of the process.

### Visual observations

In the pot (3 kg), the composition of Example 4 has a nacreous light violet appearance, the encapsulated pigments being predominantly coated with the predispersion of bismuth oxychloride.
On the hand, i.e. 0.3 g of product, the composition of Example 4 has a nacreous white-grey appearance, the encapsulated pigments being predominantly coated with the predispersion of bismuth oxychloride.
Applied to and spread on the skin, this composition imparts a luminous effect to the complexion while making it even.

In the pot (3 kg), the composition of Example 5 has a nacreous pink appearance, the encapsulated pigments being predominantly coated with the predispersion of bismuth oxychloride.
On the hand, i.e. 0.3 g of product, the composition of Example 5 has a nacreous pink appearance, the encapsulated pigments being predominantly coated with the predispersion of bismuth oxychloride.
Applied to and spread on the skin, this composition imparts a luminous effect to the complexion while making it even.

### Example 6: Composition according to the invention with a reflective particle of Mica-TiO₂ type predispersed in an ETHYLHEXYL HYDROXYSTEARATE oil

A W/O emulsion was prepared according to the protocol described in Example 1, in which the bismuth oxychloride predispersed in ethylhexyl hydroxystearate was replaced with a predispersion of nacre based on mica coated with titanium oxide in ethylhexyl hydroxystearate.

| Chemical name | % |
|---|---|
| MAGNESIUM SULFATE. 7 H₂O | 0.7 |
| DISTEARYLDIMETHYLAMMONIUM- MODIFIED HECTORITE | 0.8 |
| TALC: MICRONIZED MAGNESIUM SILICATE (PARTICLE SIZE: 5 MICRONS) (Cl: 77718) | 0.5 |
| SYNTHETIC MICA (FLUORPHLOGOPITE) (SIZE 10-50 µM) | 0.2 |
| ANATASE TITANIUM OXIDE COATED WITH | 1.8 |
| ALUMINIUM STEAROYL GLUTAMATE (97/3) (Cl: 77891) | |
| MIXTURE SYNTHETIC TITANIUM DIOXIDE LITHOL RUBINE BCA, POLYMETHYLDROGENSILOXANE (TR-50FG from TODA KOGYO) | 0.06 |
| **MICROENCAPSULATED YELLOW IRON OXIDE (YELLOWCAP1 from TAGRA BIOTECHNOLOGIES)** | **2** |
| **MICROENCAPSULATED RED IRON OXIDE (REDCAP1 from TAGRA BIOTECHNOLOGIES)** | **0.71** |
| **MICROENCAPSULATED BLACK IRON OXIDE (BLACKCAP1 from TAGRA BIOTECHNOLOGIES)** | **0.29** |
| REFINED PLANT PERHYDROSQUALENE | 1 |
| 2-ETHYLHEXYL 4-METHOXYCINNAMATE | 3 |
| **MICA-TITANIUM OXIDE (60/40) (CI: 77019 + 77891)** | **4** |
| **ETHYLHEXYL HYDROXYSTEARATE** | **4.6** |
| NYLON-12 MICROSPHERES (PARTICLE SIZE: 5 MICRONS) | 0.5 |
| PHENYLTRIMETHYLSILOXYTRISILOXANE (VISCOSITY: 20 cSt - MW: 372) | 2 |
| POLYDIMETHYLSILOXANE COMPRISING ALPHA,OMEGA-OXYETHYLENATED /- OXYPROPYLENATED GROUPS IN SOLUTION IN CYCLOPENTASILOXANE | 1 |
| OXYETHYLENATED POLYDIMETHYLSILOXANE (DP: 70 - VISCOSITY: 500 cSt) | 2 |
| POLYDIMETHYLSILOXANE 2 cSt | 21.32 |
| 1,3-BUTYLENE GLYCOL | 3 |
| DENATURED 96° ETHYL ALCOHOL | 5 |
| MICROBIOLOGICALLY CLEAN DEIONIZED WATER | 45.52 |

### Procedure

A dispersion of mica-TiO₂ in ethylhexyl hydroxystearate was prepared beforehand.

The fatty and aqueous phases were then prepared according to the protocol described in Example 1.

The dispersion of mica-TiO₂ in ethylhexyl hydroxystearate is added with stirring, after obtaining the emulsion and before integration of the encapsulated pigments.

### Visual observations

In the pot (3 kg), the composition of Example 6 has a nacreous light violet appearance, the encapsulated pigments being predominantly coated with the predispersion of bismuth oxychloride.

On the hand, i.e. 0.3 g of product, the composition of Example 6 has a nacreous white-grey appearance, the encapsulated pigments being predominantly coated with the predispersion of bismuth oxychloride.

Applied to and spread on the skin, this composition imparts a luminous effect to the complexion while making it even.

### Example 7: Composition according to the invention with Unisphere encapsulated pigments (Induchem)

| Chemical name (INCI name) | % |
|---|---|
| MAGNESIUM SULFATE. 7 H₂O | 0.70 |
| DISTEARYLDIMETHYLAMMONIUM- MODIFIED HECTORITE | 0.80 |
| TALC: MICRONIZED MAGNESIUM SILICATE (PARTICLE SIZE: 5 MICRONS) (Cl: 77718) | 0.50 |
| **MANNITOL, CELLULOSE, HYDROXYPROPYLMETHYLCELLULOSE AND TITANIUM DIOXIDE AND IRON OXIDES UNISPHERES WNRM-619 SBI (Induchem)** | **9.00** |
| REFINED PLANT PERHYDROSQUALENE | 1.00 |
| 2-ETHYLHEXYL 4-METHOXYCINNAMATE | 3.00 |
| **BISMUTH OXYCHLORIDE AND ETHYLHEXYL HYDROXYSTEARATE (TIMIRON LIQUID SILVER from Merck)** | **3.00** |
| NYLON-12 MICROSPHERES (PARTICLE SIZE: 5 MICRONS) | 0.50 |
| PHENYLTRIMETHYLSILOXYTRISILOXANE (VISCOSITY: 20 cSt - MW: 372) | 2.00 |
| POLYDIMETHYLSILOXANE COMPRISING ALPHA,OMEGA-OXYETHYLENATED /-OXYPROPYLENATED GROUPS IN SOLUTION IN CYCLOPENTASILOXANE | 1.00 |
| OXYETHYLENATED POLYDIMETHYLSILOXANE (DP: 70 - VISCOSITY: 500 cSt) | 2.00 |
| POLYDIMETHYLSILOXANE 2 cSt | 22.98 |
| 1,3-BUTYLENE GLYCOL | 3.00 |
| DENATURED 96° ETHYL ALCOHOL | 5.00 |
| MICROBIOLOGICALLY CLEAN DEIONIZED WATER | 45.52 |

Observation in bulk (in the 3 kg pot): Nacreous white, the particles coated with the nacreous liquor are camouflaged, the particles which are not coated with the nacreous liquor are visible.

### Procedure

This W/O emulsion was prepared according to the protocol described in Example 1, the only difference being that the Tagra encapsulated pigments are replaced with the Unisphere encapsulated pigments.

### Visual observations

In the pot (3 kg), the composition of Example 7 has a nacreous white appearance, the encapsulated pigments being predominantly coated with the predispersion of bismuth oxychloride.

On the hand, i.e. 0.3 g of product, the composition of Example 7 has a nacreous white appearance, the encapsulated pigments being predominantly coated with the predispersion of bismuth oxychloride.

Applied to and spread on the skin, this composition imparts a luminous effect to the complexion while making it even.

### Example 8: Composition according to the invention with encapsulated pigments of Sugar capsules© type (Daito)

| Chemical name | % |
|---|---|
| MAGNESIUM SULFATE. 7 H₂O | 0.70 |
| DISTEARYLDIMETHYLAMMONIUM- MODIFIED HECTORITE | 0.80 |
| TALC: MICRONIZED MAGNESIUM SILICATE (PARTICLE SIZE: 5 MICRONS) (Cl: 77718) | 0.50 |
| **Green Chromium oxide sugar/PMMA capsule SUGARCAPSULE MAGIC GREEN 50 ( DAITO KASEI)** | **2.00** |
| **Violet - sugar/PMMA capsule SUGARCAPSULE MAGIC VIOLET 50 ( DAITO KASEI)** | **2.00** |
| REFINED PLANT PERHYDROSQUALENE | 1.00 |
| PROTECTED 2-ETHYLHEXYL 4-METHOXYCINNAMATE | 3.00 |
| **BISMUTH OXYCHLORIDE AND ETHYLHEXYL HYDROXYSTEARATE(TIMIRON LIQUID SILVER from Merck)** | **3.00** |
| NYLON-12 MICROSPHERES (PARTICLE SIZE: 5 MICRONS) | 0.50 |
| PHENYLTRIMETHYLSILOXYTRISILOXANE (VISCOSITY: 20 cSt - MW: 372) | 2.00 |
| POLYDIMETHYLSILOXANE COMPRISING ALPHA,OMEGA-OXYETHYLENATED /-OXYPROPYLENATED GROUPS IN SOLUTION IN CYCLOPENTASILOXANE | 1.00 |
| OXYETHYLENATED POLYDIMETHYLSILOXANE (DP: 70 - VISCOSITY: 500 cSt) | 2.00 |
| POLYDIMETHYLSILOXANE 2 cSt | 27.98 |
| 1,3-BUTYLENE GLYCOL | 3.00 |
| DENATURED 96° ETHYL ALCOHOL | 5.00 |
| MICROBIOLOGICALLY CLEAN DEIONIZED WATER | 45.52 |

### Procedure

This W/O emulsion was prepared according to the protocol described in Example 1, the only difference being that the Tagra encapsulated pigments are replaced with the Sugar capsules© encapsulated pigments.

### Visual observations

In the pot (3 kg), the composition of Example 8 has a nacreous white appearance, the encapsulated pigments being predominantly coated with the predispersion of bismuth oxychloride.

On the hand, i.e. 0.3 g of product, the composition of Example 8 has a nacreous white appearance, the encapsulated pigments being predominantly coated with the predispersion of bismuth oxychloride.

Applied to and spread on the skin, this composition imparts a luminous effect to the complexion while making it even.

## Claims

1. Cosmetic composition for caring for and/or making up keratin materials, in particular the skin, comprising, in a physiologically acceptable medium:
(i) at least one encapsulated pigment; and
(ii) at least one reflective particle predispersed in at least one oil chosen from, plant oils and ester oils,
the plant oils being chosen from sweet almond oil, wheat germ oil, jojoba oil, apricot oil, soya bean oil, or canola oil, and
the ester oils being chosen from octyldodecyl neopentanoate, caprylic/capric triglycerides, pentaerythrityl tetraisostearate, diisopropyl sebacate, C₁₂-C₁₅ alkyl benzoate,
ethylhexyl ethylhexanoate or ethylhexyl hydroxystearate, and mixtures thereof.

2. Cosmetic composition according to the preceding claim, in which the encapsulated pigment is chosen from organic pigments and inorganic pigments, and mixtures thereof, preferably chosen from inorganic pigments.

3. Cosmetic composition according to either of the preceding claims, in which the inorganic pigment is chosen from metal oxides, such as a titanium oxide, zirconium oxide, cerium oxide, zinc oxide or iron oxide, ferric blue, chromium oxide and aluminium oxide, in particular chosen from titanium oxides and iron oxides, and mixtures thereof.

4. Cosmetic composition according to one of the preceding claims, in which the encapsulated pigment is chosen from:
- microcapsules constituted of jojoba esters;
- microcapsules of polymers or copolymers, such as polyacrylates or polymethacrylates, or vinyl polymers;
- microcapsules of polysaccharides, for instance cellulose derivatives, such as, for example, hydroxymethylcellulose, hydroxyethylcellulose, hydroxypropylcellulose and carboxymethylcellulose; starch; chitosan; algins; alginates; agars; agaroses; pectins; polypectates or carrageenans;
- microcapsules of polyamides;
- microcapsules of copolymers based on styrene/acrylate;
- and mixtures thereof.

5. Cosmetic composition according to one of the preceding claims, **characterized in that** the encapsulated pigment is present in a pigment active material content ranging from 0.5% to 20% by weight, in particular from 1 % to 15% by weight and more particularly from 2% to 12% by weight relative to the total weight of said composition.

6. Composition according to any one of the preceding claims, in which the reflective particles are chosen from bismuth oxychloride, nacres, and particles with a metallic glint, preferably from bismuth oxychloride and micas coated with titanium oxide, and mixtures thereof.

7. Composition according to the preceding claim, in which the reflective particles are predispersed in at least one oil chosen from 2-ethylhexyl hydroxystearate.

8. Cosmetic composition according to any one of the preceding claims, in which the reflective particles are chosen from bismuth oxychloride and micas coated with titanium oxide, predispersed in at least 2-ethylhexyl hydroxystearate.

9. Composition according to any one of the preceding claims, **characterized in that** the reflective particles are predispersed in at least one oil in a reflective particles/oil weight ratio of greater than or equal to 1, in particular ranging from 2 to 5, especially from 2 to 3.

10. Cosmetic composition according to any one of the preceding claims, **characterized in that** the weight ratio between the encapsulated pigments and the reflective particles will range from 3.33 x 10⁻⁴ to 10, especially from 3.33 x 10⁻⁴ to 8, in particular from 3.33 x 10⁻⁴ to 6, or even from 3.33 x 10⁻⁴ to 4.3.

11. Composition according to any one of the preceding claims, **characterized in that** it is in the form of a fluid composition comprising at least one oily fatty phase, in particular in the form of an oil-in-water (O/W) emulsion, a water-in-oil (W/O) emulsion or a multiple emulsion, preferably a water-in-oil (W/O) emulsion.

12. Composition according to one of the preceding claims, **characterized in that** it also contains at least one additional ingredient chosen from silicone oils or hydrocarbon-based oils, surfactants, fillers, non-encapsulated additional colorants, and mixtures thereof.

13. Cosmetic process for caring for and/or making up keratin materials, comprising the application to said keratin materials, in particular to the skin, of a composition as defined in any one of the preceding claims.

14. Process for preparing a cosmetic composition for caring for and/or making up keratin materials, in particular the skin, comprising the addition, to a physiologically acceptable medium comprising (i) at least encapsulated pigments, of (ii) at least reflective particles in the form of a predispersion in an oil chosen from
- plant oils selected from sweet almond oil, wheat germ oil, jojoba oil, apricot oil, soya bean oil, and canola oil;
- esters selected from octyldodecyl neopentanoate, caprylic/capric triglyerides, pentaerythrityl tetraisostearate, diisopropyl sebacate, C₁2-C₁₅ alkyl benzoate, ethylhexyl ethylhexanoate and ethylhexyl hydroxystearate,
- and mixtures thereof.

15. Use of reflective particles predispersed in an oil chosen from plant oils, esters and mixtures thereof, in a cosmetic composition comprising encapsulated pigments, as an agent intended for reducing the visibility of the encapsulated pigments in the bulk composition,
the plant oils being chosen from sweet almond oil, wheat germ oil, jojoba oil, apricot oil, soya bean oil or canola oil, and the ester being chosen from octyldodecyl neopentanoate, caprylic/capric triglycerides, pentaerythrityl tetraisostearate, diisopropyl sebacate, C₁₂-C₁₅ alkyl benzoate, ethylhexyl ethylhexanoate or ethylhexyl hydroxystearate, and mixtures thereof.

## Patentansprüche

1. Kosmetische Zusammensetzung zur Pflege für und/oder zum Makeup von Keratinmaterialien, insbesondere der Haut, umfassend in einem physiologisch akzeptablen Medium:
(i) mindestens ein eingekapseltes Pigment; und
(ii) mindestens ein reflektierendes Teilchen, das in mindestens einem Öl, das aus Pflanzenölen und Esterölen ausgewählt ist, vordispergiert ist,
wobei die Pflanzenöle aus Süßmandelöl, Weizenkeimöl, Jojobaöl, Aprikosenöl, Sojabohnenöl oder Rapsöl ausgewählt sind, und
die Esteröle aus Octyldodecylneopentanoat, Caprylsäure-/Caprinsäuretriglyceriden, Pentaerythrityltetraisostearat, Diisopropylsebacat, C₁₂-C₁₅-Alkylbenzoat, Ethylhexylethylhexanoat oder Ethylhexylhydroxystearat und Gemischen davon ausgewählt sind.

2. Kosmetische Zusammensetzung nach dem vorhergehenden Anspruch, bei der das eingekapselte Pigment aus organischen Pigmenten und anorganischen Pigmenten und Gemischen davon ausgewählt ist, und vorzugsweise aus anorganischen Pigmenten ausgewählt ist.

3. Kosmetische Zusammensetzung nach einem der vorhergehenden Ansprüche, bei der das anorganische Pigment aus Metalloxiden, wie Titanoxid, Zirkonoxid, Ceroxid, Zinkoxid oder Eisenoxid, Eisenblau, Chromoxid und Aluminiumoxid ausgewählt ist, und insbesondere aus Titanoxiden und Eisenoxiden und Gemischen davon ausgewählt ist.

4. Kosmetische Zusammensetzung nach einem der vorhergehenden Ansprüche, bei der das eingekapselte Pigment ausgewählt ist aus:
- Mikrokapseln, die aus Jojoba-Estern gebildet sind;
- Mikrokapseln von Polymeren oder Copolymeren wie Polyacrylaten oder Polymethacrylaten oder Vinylpolymeren;
- Mikrokapseln von Polysacchariden, beispielsweise Cellulosederivaten, wie z.B. Hydroxymethylcellulose, Hydroxyethylcellulose, Hydroxypropylcellulose und Carboxymethylcellulose; Stärke; Chitosan; Alginen; Alginaten; Agar; Agarosen; Pektinen; Polypektaten oder Carrageenanen;
- Mikrokapseln von Polyamiden;
- Mikrokapseln von Copolymeren auf der Basis von Styrol/Acrylat;
- und Gemischen davon.

5. Kosmetische Zusammensetzung nach einem der vorhergehenden Ansprüche, **dadurch gekennzeichnet, dass** das eingekapselte Pigment in einem Pigment-Wirkstoffgehalt im Bereich von 0,5 bis 20 Gew.-%, insbesondere von 1 bis 15 Gew.-% und ganz besonders von 2 bis 12 Gew.-%, bezogen auf das Gesamtgewicht der Zusammensetzung, zugegen ist.

6. Zusammensetzung nach einem der vorhergehenden Ansprüche, bei der die reflektierenden Teilchen aus Wismutoxychlorid, Perlmutt und Teilchen mit einem metallischen Glanz, vorzugsweise aus Wismutoxychlorid und mit Titanoxid beschichteten Glimmern, und Gemischen davon ausgewählt sind.

7. Zusammensetzung nach dem vorhergehenden Anspruch, bei der die reflektierenden Teilchen in mindestens einem Öl, ausgewählt aus 2-Ethylhexylhydroxystearat, vordispergiert sind.

8. Kosmetische Zusammensetzung nach einem der vorhergehenden Ansprüche, bei der die reflektierenden Teilchen aus Wismutoxychlorid und mit Titanoxid beschichteten Glimmern ausgewählt sind, und in mindestens 2-Ethylhexylhydroxystearat vordispergiert sind.

9. Zusammensetzung nach einem der vorhergehenden Ansprüche, **dadurch gekennzeichnet, dass** die reflektierenden Teilchen in mindestens einem Öl in einen Gewichtsverhältnis von reflektierenden Teilchen/Öl von größer oder gleich 1, insbesondere im Bereich von 2 bis 5, insbesondere von 2 bis 3, vordispergiert sind.

10. Kosmetische Zusammensetzung nach einem der vorhergehenden Ansprüche, **dadurch gekennzeichnet, dass** das Gewichtsverhältnis zwischen den eingekapselten Pigmenten und den reflektierenden Teilchen von 3,33 x 10-⁴ zu 10, insbesondere von 3,33 x 10⁻⁴ zu 8, insbesondere von 3,33 x 10⁻⁴ zu 6 oder sogar von 3,33 x 10⁻⁴ zu 4,3 reicht.

11. Zusammensetzung nach einem der vorhergehenden Ansprüche, **dadurch gekennzeichnet, dass** sie in Form einer Fluidzusammensetzung vorliegt, die mindestens eine ölige Fettphase, insbesondere in der Form einer Öl-in-Wasser-(O/W)-Emulsion, einer Wasser-in-öl-(W/O)-Emulsion oder einer Mehrfachemulsion, vorzugsweise einer Wasser-in-Öl-(W/O)-Emulsion, umfasst.

12. Zusammensetzung nach einem der vorhergehenden Ansprüche, **dadurch gekennzeichnet, dass** sie auch mindestens einen zusätzlichen Bestandteil enthält, der aus Silikonölen oder Ölen auf Kohlenwasserstoffbasis, oberflächenaktiven Mitteln, Füllstoffen, nicht eingekapselten zusätzlichen Farbstoffen und Gemischen davon ausgewählt ist.

13. Kosmetisches Verfahren zur Pflege für und/oder zum Makeup von Keratinmaterialien, umfassend die Ausbringung einer Zusammensetzung nach einem der vorhergehenden Ansprüche auf die Keratinmaterialien, insbesondere auf die Haut.

14. Verfahren zur Herstellung einer kosmetischen Zusammensetzung zur Pflege für und/oder zum Makeup von Keratinmaterialien, insbesondere der Haut, umfassend die Zugabe zu einem physiologisch akzeptablen Medium, umfassend (i) mindestens eingekapselte Pigmente, von (ii) mindestens reflektierenden Partikeln in der Form einer Vordispersion in einem Öl, ausgewählt aus
- Pflanzenölen, ausgewählt aus Süßmandelöl, Weizenkeimöl, Jojobaöl, Aprikosenöl, Sojabohnenöl und Rapsöl;
- Estern, ausgewählt aus Octyldodecylneopentanoat, Caprylsäure-/Caprinsäuretriglyceriden, Pentaerythrityltetraisostearat, Diisopropylsebacat, C₁₂-C₁₅-Alkylbenzoat, Ethylhexylethylhexanoat und Ethylhexylhydroxystearat,
- und Gemischen davon.

15. Verwendung reflektierender Teilchen, die in einem Öl, das aus Pflanzenölen, Estern und Gemischen davon ausgewählt ist, vordispergiert ist, in einer kosmetischen Zusammensetzung, umfassend eingekapselte Pigmente, als Mittel, das zur Verringerung der Sichtbarkeit der eingekapselten Pigmente in der Massezusammensetzung vorgesehen ist, wobei die Pflanzenöle aus Süßmandelöl, Weizenkeimöl, Jojobaöl, Aprikosenöl, Sojabohnenöl, oder Rapsöl ausgewählt sind, und die Ester aus Octyldodecylneopentanoat, Caprylsäure-/Caprinsäuretriglyceriden, Pentaerythrityltetraisostearat, Diisopropylsebacat, C₁₂-C₁₅-Alkylbenzoat, Ethylhexylethylhexanoat oder Ethylhexylhydroxystearat und Gemischen davon ausgewählt sind.

## Revendications

1. Composition cosmétique de soin et/ou de maquillage des matières kératiniques, en particulier de la peau comprenant, dans un milieu physiologiquement acceptable :
(i) au moinsun pigment encapsulé : et
(ii) au moins une particule réfléchissante pré-dispersée dans au moins une huile choisie parmi les huiles végétales et les huiles esters,
les huiles végétales étant choisies parmi l'huile d'amande douce, l'huile de germe de blé, l'huile de jojoba, l'huile d'abricot, l'huile de soja ou l'huile de canola, et les huiles d'ester étant choisies parmi le néopentanoate d'octyldodécyle, les triglycérides capryliques/capriques, le tétraisostéarate de pentaérythrityle, le sébacate de diisopropyle, le benzoate de C₁₂-C₁₅-alkyle, l'éthylhexanoate d'éthylhexyle ou l'hydroxystéarate d'éthylhexyle, et des mélanges de ceux-ci.

2. Composition cosmétique selon la revendication précédente, dans laquelle le pigment encapsulé est choisi parmi les pigments organiques, et les pigments inorganiques, et leurs mélanges, de préférence choisi parmi les pigments inorganiques.

3. Composition cosmétique selon l'une des revendications précédentes, dans laquelle le pigment inorganique est choisi parmi les oxydes métalliques, tels qu'un oxyde de titane, un oxyde de zirconium, un oxyde de cérium, un oxyde de zinc ou un oxyde de fer, le bleu ferrique, un oxyde de chrome et un oxyde d'aluminium, en particulier choisi parmi les oxydes de titane et les oxydes de fer, et leurs mélanges.

4. Composition cosmétique selon l'une des revendications précédentes, dans laquelle le pigment encapsulé est choisi parmi :
- des microcapsules constituées d'esters de - jojoba ;
- des microcapsules de polymères ou de copolymères, tels que les polyacrylates ou polyméthacrylates, ou les polymères vinyliques ;
- des microcapsules de polysaccharides comme les dérivés de cellulose, telles que par exemple l'hydroxy méthylcellulose, l'hydroxy éthylcellulose, l'hydroxy propyl cellulose et la carboxylméthylcellulose ; l'amidon ; le chitosan ; les algines ; les alginates ; les agars ; les agaroses ; les pectines ; les polypectates ou les carraghénanes ;
- des microcapsules de polyamides ;
- des microcapsules de copolymères à base de styrène/acrylate ;
- et leurs mélanges.

5. Composition cosmétique selon l'une des revendications précédentes, dans laquelle le pigment encapsulé est présent en une teneur en matière active de pigment allant de 0,5% à 20 % en poids, en particulier de 1 % à 15 % en poids, et plus particulièrement de 2 % à 12 % en poids, par rapport au poids total de ladite composition.

6. Composition selon l'une quelconque des revendications précédentes, dans laquelle les particules réfléchissantes sont choisies parmi l'oxychlorure de bismuth, les nacres, et les particules à reflet métallique, de préférence parmi l'oxychlorure de bismuth et les micas recouverts d'oxyde de titane, et leurs mélanges.

7. Composition selon la revendication précédente, dans laquelle les particules réfléchissantes sont pré-dispersées dans au moins une huile choisie parmi l'hydroxystéarate de 2-éthylhexyle.

8. Composition cosmétique selon l'une quelconque des revendications précédentes, dans laquelle les particules réfléchissantes sont choisies parmi l'oxychlorure de bismuth et les micas recouverts d'oxyde de titane pré-dispersés dans au moins de l'hydroxystéarate de 2-éthylhexyle.

9. Composition selon l'une quelconque des revendications précédentes, **caractérisée en ce que** les particules réfléchissantes sont pré-dispersées dans au moins une huile dans un rapport pondéral particules réfléchissantes/huile supérieur ou égal à 1, en particulier allant de 2 à 5, notamment de 2 à 3.

10. Composition cosmétique selon l'une quelconque des revendications précédentes, **caractérisée en ce que** le rapport pondéral entre les pigments encapsulés et les particules réfléchissantes va de 3.33*10⁻⁴ à 10, notamment de 3.33*10⁻⁴ à 8, en particulier de 3.33*10⁻⁴ à 6, voire de 3.33*10⁻⁴ à 4.3.

11. Composition selon l'une quelconque des revendications précédentes, **caractérisée en ce qu'**elle est sous la forme d'une composition fluide comprenant au moins une phase grasse huileuse, en particulier sous la forme d'une émulsion huile-dans-eau (H/E), d'une émulsion eau-dans-huile (E/H) ou d'une émulsion multiple, de préférence une émulsion eau-dans-huile (E/H).

12. Composition selon l'une des revendications précédentes, **caractérisée en ce qu'**elle contient en outre au moins un ingrédient additionnel choisi parmi les huiles siliconées ou hydrocarbonées, les tensioactifs, les charges, les matières colorantes additionnelles non encapsulées, et leurs mélanges.

13. Procédé cosmétique de soin et/ou de maquillage des matières kératiniques comprenant l'application, sur lesdites matières kératiniques, en particulier sur la peau, d'une composition telle que définie dans l'une quelconque des revendications précédentes.

14. Procédé de préparation d'une composition cosmétique de soin, et/ou de maquillage des matières kératiniques, en particulier de la peau, comprenant l'addition, à un milieu physiologiquement acceptable comprenant (i) au moins des pigments encapsulés, (ii) d'au moins des particules réfléchissantes sous la forme d'une pré-dispersion dans une huile choisie parmi
- les huiles végétales choisies parmi l'huile d'amande douce, l'huile de germe de blé, l'huile de jojoba, l'huile d'abricot, l'huile de soja et l'huile de canola ;
- les esters choisis parmi le néopentanoate d'octyldodécyle, les triglycérides capryliques/capriques, le tétraisostéarate de pentaérythrityle, le sébacate de diisopropyle, le benzoate de C₁₂-C₁₅-alkyle, l'éthylhexanoate d'éthylhexyle et l'hydroxystéarate d'éthylhexyle,
- et leurs mélanges.

15. Utilisation de particules réfléchissantes pré-dispersées dans une huile choisie parmi les huiles végétales, les esters et leurs mélanges, dans une composition cosmétique comprenant des pigments encapsulés, comme agent destiné à diminuer la visibilité des pigments encapsulés dans la composition en masse,
les huiles végétales étant choisies parmi l'huile d'amande douce, l'huile de germe de blé, l'huile de jojoba, l'huile d'abricot, l'huile de soja ou l'huile de canola, et l'ester étant choisi parmi le néopentanoate d'octyldodécyle, les triglycérides capryliques/capriques, le tétraisostéarate de pentaérythrityle, le sébacate de diisopropyle, le benzoate de C₁₂-C₁₅-alkyle, l'éthylhexanoate d'éthylhexyle ou l'hydroxystéarate d'éthylhexyle, et des mélanges de ceux-ci.
